# EUROPEAN PATENT APPLICATION

(11) **EP 4 666 946 A1**
(43) Date of publication of application: **24.12.2025**
(21) Application number: 24756228.3
(22) Date of filing: 07.02.2024
(51) Int. Cl.: A61B 5/256, A61B 5/251, A61B 5/291, A61B 5/369, A61B 5/386, A61N 1/36, A61N 1/04, A61B 5/282

(54) **ELECTRODE SEAT STRUCTURE AND ELECTROENCEPHALOGRAM CAP**

(30) Priority: 16.02.2023 CN 202320240075 U; 16.02.2023 CN 202320235353 U
(71) Applicant: Neuracle Technology (Shanghai) Co., Ltd., Shanghai 200120 (CN)
(72) Inventor: WANG, Zhen, hangzhou, Jiangsu 213000 (CN)
(74) Representative: Sun, Yanan
(86) International application number: PCT/CN2024/076624
(87) International publication number: WO 2024/169855

(57) **Abstract**

The present disclosure relates to electrode holders and discloses an electrode holder structure and an electroencephalogram (EEG) cap, comprising a base, where multiple clamping jaws are disposed circumferentially, thereby enclosing an accommodation area; an electrode holder, disposed in the accommodation area and fixedly clamped by multiple clamping jaws to form the electrode holder structure. Multiple clamping jaws are disposed circumferentially around the base, and at least one electrode holder is disposed in the multiple layers of accommodation areas formed by multiple clamping jaws, so as to realize stacking disposal of the electrode holder and the seat and improve the entire generality.

## Description

### CROSS-REFERENCE TO THE RELATED APPLICATIONS

This application claims priority to Chinese Patent Application No. 202320240075.1 filed on February 16, 2023 and Chinese Patent Application No. 202320235353.4 filed on February 16, 2023, the entire contents of which are incorporated herein by reference.

### TECHNICAL FIELD

The present disclosure relates to the field of electroencephalogram caps and in particular to an electrode holder structure and an electroencephalogram (EEG) cap.

### BACKGROUND

The electroencephalogram (EEG) is a safe non-invasive and low-cost detection technology capable of directly reflecting the activities of brain neurons. In the clinical applications, the EEG technology is based on the recorded physiological and pathological information to be widely applied to the auxiliary diagnoses of the diseases of central nervous systems and the mental diseases, for example, to the detection of epilepsy, Parkinson's disease and brain death etc.

In the relates arts, the snapping solution of the electrode holder structure and the electrode pad employed in the EEG cap is a single-layer snapping with one snap, bringing inconvenience to angle switching. Furthermore, in the related arts, a wet electrode EEG cap with a detachable electrode and cap-connected syringe is usually adopted. The EEG cap includes electrode bodies. On the top of the electrode bodies is further provided with a protruding fixing bolt, and the electrode is mounted onto the corresponding electrode body through the protruding fixing bolt. Also, the protruding fixing bolt on one electrode body is correspondingly provided with one electrode, that is, only one electrode pad is disposed on one electrode holder. Further, the ordinary electrode holder structure is complex and difficult to mount and dismount, and the wiring position therein is single and requires separate adjustment each time.

### SUMMARY

In order to solve the technical problems, the present disclosure provides an electrode holder structure to facilitate switching mounting angles of the electrode holder and improve the flexibility of wire arrangement of the electrode holder.

The present disclosure provides an electrode holder structure with combined use of a stimulating electrode and a collecting electrode, which includes:
a base, where multiple clamping jaws are disposed around the base, thereby enclosing an accommodation area;
an electrode holder, disposed in the accommodation area and fixedly clamped by multiple clamping jaws to form the electrode holder structure.

Other features and advantages of the present disclosure will be set forth in the following description, and further become partially apparent from the description or known by carrying out the present disclosure. The objects and other advantages of the present disclosure are implemented and obtained by the structures specially indicated in the description and drawings.

In order to make the objects, features and advantages of the present disclosure clearer and more intelligible, detailed descriptions are made below by way of preferred embodiments and drawings.

### BRIEF DESCRIPTION OF THE DRAWINGS

In order to more clearly describe the specific embodiments in the present disclosure or the technical solutions in the prior arts, brief introduction will be made to the drawings required for description of the specific embodiments or the prior arts. Apparently, the drawings described hereunder are some embodiments of the present disclosure. Those skilled in the arts can also obtain other drawings based on these drawings without carrying out creative work.
FIG. 1 is a schematic diagram illustrating an assembling structure of an electrode holder structure according to a first embodiment of the present disclosure.

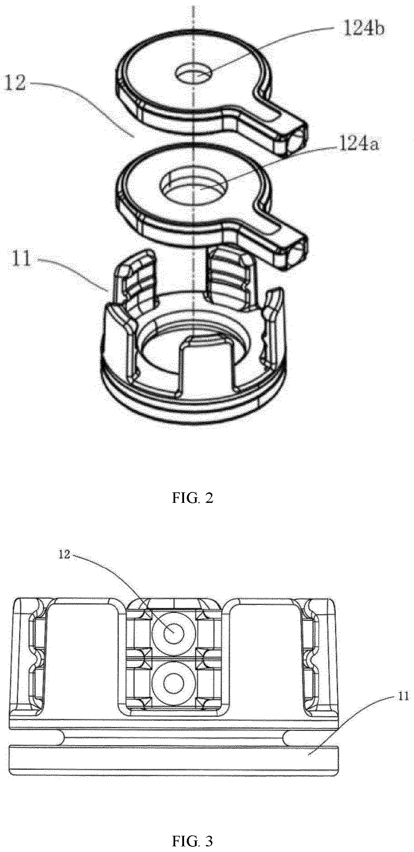

FIG. 2 is an exploded view illustrating an electrode holder structure with combined use of a stimulating electrode and a collecting electrode according to a first embodiment of the present disclosure.
FIG. 3 is a side view illustrating an electrode holder structure based on a seat of an EEG cap according to a first embodiment of the present disclosure.
FIG. 4 is a structural schematic diagram illustrating a base of an electrode holder structure with combined use of a stimulating electrode and a collecting electrode according to a first embodiment of the present disclosure.
FIG. 5 is a structural schematic diagram illustrating an electrode holder of an electrode holder structure with combined use of a stimulating electrode and a collecting electrode according to a first embodiment of the present disclosure.
FIG. 6 is an exploded view illustrating an electrode holder structure according to a second embodiment of the present disclosure.
FIG. 7 is an assembling diagram illustrating a base and a second electrode holder of an electrode holder structure according to a second embodiment of the present disclosure.
FIG. 8 is an assembling diagram illustrating a base and a first electrode holder of an electrode holder structure according to a second embodiment of the present disclosure.
FIG. 9 is a an assembling diagram illustrating a base, a first electrode holder and a second electrode holder of an electrode holder structure according to a second embodiment of the present disclosure.
FIG. 10 is a structural schematic diagram illustrating a base of an electrode holder structure according to a second embodiment of the present disclosure.
FIG. 11 is a structural schematic diagram illustrating a first electrode holder of an electrode holder structure according to a second embodiment of the present disclosure.
FIG. 12 is a structural schematic diagram illustrating a second electrode holder of an electrode holder structure according to a second embodiment of the present disclosure.
FIG. 13 is a structural schematic diagram illustrating a bottom of a base of an electrode holder structure according to a second embodiment of the present disclosure.
FIG. 14 is a structural schematic diagram illustrating a bottom of a first electrode holder of an electrode holder structure according to a second embodiment of the present disclosure.
FIG. 15 is a structural schematic diagram illustrating a bottom of a second electrode holder of an electrode holder structure according to a second embodiment of the present disclosure.

In the drawings: base 11, 21; electrode holder 12, 22, 23; clamping jaw 111, 214, 223; seat 112; clamping groove 1111, 2142, 2232; protrusion 2141, 2231; first through hole 115, 215; second through hole 124a, 224; third through hole 124b, 234; circular sheet 113; upper-layer circular sheet 212; upperlower-layer circular sheet 211; mounting gap 114, 213; circular portion 121, 221, 231; tail portion 122, 222, 232; avoiding portion 123; channel structure 125; hole 2221, 2321; flange 2311; clamping strip 225, 235; stop strip 233, 226.

### DETAILED DESCRIPTION OF THE EMBODIMENTS

In order to make the objects, technical solutions and advantages of the embodiments of the present disclosure clearer, the technical solutions of the present disclosure will be clearly and fully described below in combination with drawings. Apparently, the embodiments described herein are only some embodiments of the present disclosure rather than all embodiments. All other embodimnets obtained by those skilled in the arts based on these embodiments of the present disclosure without carrying creative work shall fall within the scope of protection of the present disclosure.

At least one embodiment provides an electrode holder structure for combined use of an stimulating electrode and a collecting electrode. The electrode holder structure includes a base (11, 21), where multiple clamping jaws 111 are disposed in a circumferential direction of the base (in the following specific implementation process, numerals corresponding to a first clamping jaw 214 and a clamping jaw 223), and all the jaws are enclosed to form an accommodation area; and an electrode holder, disposed inside the accommodation area and fixedly clamped by multiple clamping jaws to form the electrode holder structure.

When there is one electrode holder, the base further includes a seat and each clamping jaw is disposed circumferentially on an edge of the seat; one clamping groove is disposed on an inner side of the clamping jaws, and an accommodation area is formed among the clamping grooves to clamp the corresponding electrode holder.

When there are at least two electrode holders, the two electrode holders are stimulating electrode holders (hereinafter referred to as first electrode holder) or collecting electrode holders (hereinafter referred to as second electrode holder); or the simulating electrode holder and the collecting electrode holder are used in combination. The simulating electrode holder and the collecting electrode holder are sequentially disposed above the base.

The base further includes a seat and each clamping jaw is circumferentially disposed on a edge of the seat. Multiple clamping grooves are disposed up and down on an inner side of the clamping jaws (in some embodiments, the clamping grooves are also referred to as protrusion 1111, and in the specific implementation process, numerals correspond to a first clamping groove 2142 and a second clamping groove 2232), and multiple layers of accommodation areas are formed among the multiple clamping grooves (in some embodiments, also referred to as mounting positions) so that corresponding electrode holders can be stacked and clamped up and down.

In a case of the combination of the simulating electrode holder and the collecting electrode holder, the simulating electrode holder and the collecting electrode holder are sequentially disposed above the base, and the clamping jaws of the base are provided with clamping grooves for forming a lower-layer accommodation area to fixedly clamp the stimulating electrode holder; and the stimulating electrode holder is provided with multiple clamping jaws and a protrusion is disposed on an inner side of the multiple clamping jaws (in the specific implementation process, numerals correspond to a first protrusion 2141 and a second protrusion 2231) to form clamping grooves for forming an upper-layer accommodation area, so as to fixedly clamp the collecting electrode holder.

There are two electrode holders; and a middle part of the base is hollowed to form a first through hole (in the specific implementation process, numerals correspond to a first thorough hole 115 and a first through hole 215) with a diameter of φ1.

A middle part of the electrode holder in the lower-layer accommodation area is hollowed to form a second through hole A (in the specific implementation process, numerals correspond to a second through hole 124a and a second through hole 224) with a diameter of φa. A middle part of the electrode holder in the upper-layer accommodation area is hollowed to form a third through hole B (in the specific implementation process, numerals correspond to a third through hole B124b and a third through hole 234) with a diameter of φb. The first through hole, the second through hole and the third through hole are all disposed as concentric, and φ1>φa>φb.

The seat includes multiple layers of stacked circular sheets 113 and a mounting gap fitted with the EEG cap is disposed between the multiple layers of circular sheets.

The electrode holder includes a circular portion 121 (in the specific implementation process, numerals correspond to a first circular portion 221 and a second circular portion 231) and a tail portion (in the specific implementation process, numerals correspond to a first tail portion 222 and a second tail portion 232); the tail portion extends outwardly from the circular portion in a radial direction; and the tail portion extends out from a gap between adjacent clamping jaws.

The top of the circular portion is disposed as open; and a side of the circular portion is further provided with multiple avoiding portions which are flat planar structures.

A cover plate is provided on the top of the tail portion and forms a channel structure together with a shell of the tail portion.

The base includes an upper-layer circular sheet and a lower-layer circular sheet; a mounting gap is disposed between the upper-layer circular sheet and the lower-layer circular sheet.

The multiple clampling jaws are disposed in a spacing of same angle, and the clamping jaws corresponding to the upper-layer and lower-layer accommodation areas are arranged staggeredly.

The electrode holder includes a circular portion and a tail portion; the tail portion extends out from the circular portion in a radial direction, a bottom of the side of the circular portion extends outwardly to form a flange 2311 which is fitted with the clamping grooves in the accommodation area; and, the side of the circular portion extends outwardly to form multiple stop strips (in the specific implementation process, numerals correspond to a stop strip 233 and a stop strip 226), and an opening is opened on the bottom of the circular portion and a step surface is disposed on a circumference of the opening.

The electrode holder further includes a recessed area; the recessed area is in a middle part of the circular portion and extends to the tail portion. A hole is opened on the tail portion (in the specific implementation process, numerals correspond to a first hole 2221 and a second hole 2321), and the hole is in communication with the recessed area; a clamping strip is further disposed inside the recessed area of the tail portion (in the specific implementation process, numerals correspond to a first clamping strip 225 and a second clamping strip 235).

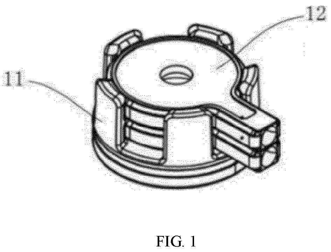

In the electrode holder structure provided in some embodiments, as shown in FIG. 1, a base 11 and at least one electrode holder 12 are included, and the entire base is formed integrally. The base 11 includes a seat 112 and multiple clamping jaws 111 disposed in a spacing along the circumference of the seat 112. The multiple clamping jaws 111 are enclosed to form an accommodation area to accommodate the electrode holder 12. After the electrode holder 12 is placed inside the accommodation area, a circumferential side of the electrode holder 12 can be abutted against an inner side of the clamping jaws 111 so as to limit the mounted electrode holder 12 with the multiple clamping jaws 111.

In order to facilitate smoothly placing the electrode holder 12 into the base 11, the base 11 and the electrode holder 12 can be made from a deformable plastic material. During mounting, the electrode holder 12 is pressed into the accommodation area so that the circumferential side of the electrode holder 12 can be fitted between the protrusions 1111 disposed on the clamping jaws 111, so as to complete the mounting of the electrode holder 12 and the base 11.

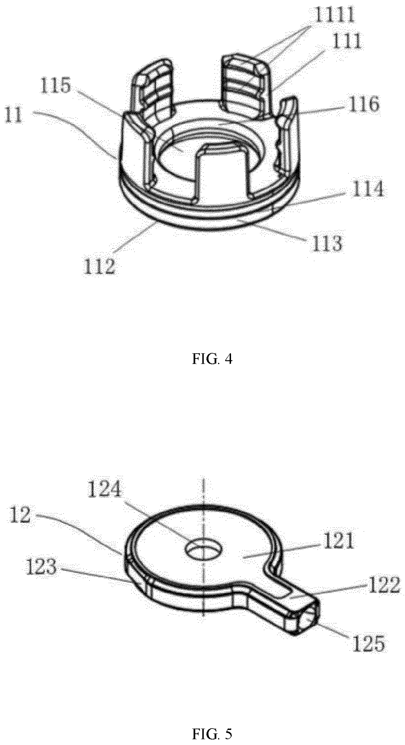

As shown in FIG. 4, multiple protrusions 1111 are further disposed on a side of each clamping jaw 111 abutted against the electrode holder 12, and the multiple protrusions 1111 are equidistantly and vertically disposed on each clamping jaw 111 to enable the accommodation area to be horizontal and adapt to a flat surface of the electrode holder 12. The clamping jaws 111 on a same height plane can divide the accommodation area into multiple accommodation areas for placing single electrode holders 12. Multiple protrusions 1111 can be disposed on each clamping jaw 111. When two protrusions 1111 are disposed on each clamping jaw 111, the accommodation area can be divided into two accommodation areas of the electrode holder 12. Also, more protrusions 1111 can be disposed, for example, three protrusions 1111 can be disposed so that the accommodation area can be divided into three accommodation areas of the electrode holder 12; four protrusions 1111 can also be disposed so that the accommodation area can be divided into four accommodation areas of the electrode holder 12 and so on. The specific number of the protrusions can be determined based on actual requirements.

Multiple electrode holders 12 can be sequentially mounted into each accommodation area or separately mounted into each accommodation area. For example, multiple protrusions 1111 divide the accommodation area into two accommodation areas of electrode holder 12 and the two accommodation areas are arranged up and down. At this time, the first electrode holder 12 can be firstly mounted onto the first accommodation area near the seat 112 and then the second electrode holder 12 is mounted onto the second accommodation area near the first electrode holder 12; or the first electrode holder 12 or the second electrode holder 12 can be directly mounted onto the first accommodation area or the second accommodation area.

The seat 112 includes multiple layers of stacked circular sheets 113 and a mounting gap 114 is disposed between the multiple layers of circular sheets 113 so as to fit with the EEG cap and realize detachable connection of the base 11 and the EEG cap. The number of the circular sheets 113 included in the seat 112 is set to 2 herein, and can also be set to three or four or the like. So, the number of the stacked circular sheets 113 is not limited herein.

More specifically, a middle part of multiple layers of stacked circular sheets 113 is hollowed to form a first through hole 115 with a diameter less than a diameter of the accommodation area formed by multiple clamping jaws 111. Furthermore, a chamfer 116 is further opened on an inner circumferential side of the first through hole 115 so as to help the use of a conductive paste.

As shown in FIG. 5, the electrode holder 12 is a hollow shell and each electrode holder 12 includes a circular portion 121 and a tail portion 122. The tail portion 122 extends in a radial direction of the circular portion 121. A middle part of the circular portion 121 is hollowed to form a second through hole 124. Further, no cover plate is provided on the top of the circular portion 121 to help mount an electrode pad. A cover plate is provided on the tail portion 122 of the electrode holder 12, and forms a channel structure 125 for accommodating a lead wire of the electrode pad together with the shell of the tail portion 122, so as to lead out the lead wire of the electrode pad through the channel structure 125.

The tail portion 122 of the electrode holder 12 can run through a gap structure formed between spaced clamping jaws 111. Herein, five clamping jaws 111 are disposed in a spacing in a circumferential direction of the seat 112 and therefore five gap structures can also be formed. The five gap structures are also distributed in the circumferential direction of the seat 112. So, when the electrode holder 12 is mounted to the seat 112, the tail portion 122 of the electrode holder 12 can face a different direction so that the electrode holder 12 can switch in five positional directions freely and the mounting angle of the electrode holder 2 can be freely adjusted, helping wiring mounting.

An avoiding portion 123 is further disposed on a side of each electrode holder 12. The avoiding portion 123 is a flat planar structure formed by cutting the side of the circular portion 121. When the electrode holder 12 is placed on the seat 112, the avoiding portion 123 disposed on the side of the circular portion 121 may not snap-fit with the corresponding clamping jaws 111 and the number of the clamping jaws snap-fitting with the side of the circular portion 121 can thus be reduced based on actual requirements, bringing convenience to mounting and dismounting. For example, when five clamping jaws are used for limitation and two avoiding portions 123 are disposed on the side of the circular portion 121, only three clamping jaws are abutted against the electrode holder, which not only satisfies the limitation requirements but also helps mounting and dismounting of the electrode holder.

When the electrode holders are of same kind or type, for example, when they are stimulating electrode holders or collecting electrode holders, the number of the electrode holders can be set to one or more. The electrode holders of same kind are stacked in the corresponding accommodation areas up and down without given placement sequence.

When the electrode holders are of different kinds or different types, the number of the electrode holders is at least two. When the stimulating electrode holder and the collecting electrode holder are used in combination, the electrode holder mounted at bottom is the stimulating electrode holder for mounting a stimulating electrode; at least one collecting electrode holder for mounting a collecting electrode (e.g. wet electrode) is sequentially stacked above the stimulating electrode holder. In this way, the convenience of combined use of the stimulating electrode and the collecting electrode can be improved for they can be directly covered on each other. A second through hole A124a is opened on the stimulating electrode holder and a second through hole B124b is opened on the collecting electrode holder. The second through hole A124a opened on the stimulating electrode holder is larger in size than the second through hole B124b opened on the collecting electrode holder. Thus, EEG paste can be filled up from bottom up to ensure up-down communication. When the circular portion 121 of the electrode holder 12 is placed into the accommodation area, since the seat 112 has the largest diameter, the first through hole 115 opened on the seat 112 is larger in diameter than the second through hole 124 opened in the middle part of the first-placed electrode holder. At this time, observed from the electrode holder 12 to the seat 112, the hole diameter gradually increases so that the conductive paste placed inside is cone-shaped and the conductive paste is in contact with the electrode pad inside the electrode holder 12, so as to improve the entire conductivity. Of course, the through holes of different diameters can be opened on different electrode holders 12. During mounting process, the electrode holder 12 with the largest through hole is disposed at bottom and the electrode holders 12 with smaller through holes can be stacked in sequence so that the finally-stacked through holes are in communication with each other to form a cone shape. Furthermore, through holes opened on the electrode holders 12 is disposed concentric with the through hole opened on the seat 112.

Multiple clamping jaws are disposed in the circumferential direction on the base and at least one electrode holder is disposed inside the accommodation area formed by multiple clamping jaws. The side of the electrode holder can be abutted against multiple clamping jaws so as to realize the fixed mounting of the electrode holder and the seat. Further, the accommodation area formed by multiple clamping jaws can accommodate multiple electrode holders or single electrode holder, so as to increase the entire general adaptation and reduce space occupation. Due to spaced disposal of multiple clamping jaws, at least one electrode holder can be mounted into the accommodation area at a different mounting angle, and its mounting angle is adjustable.

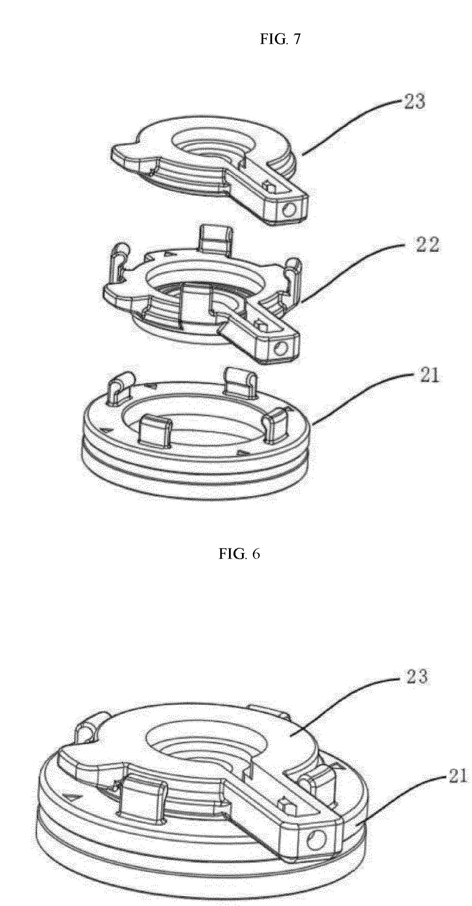

In the electrode holder structure provided in some embodiments, as shown in FIG. 6, the electrode holder structure includes a base 21 and an electrode holder. Multiple first clamping jaws 214 are disposed on the base 21 and the multiple first clamping jaws 214 are enclosed to form an accommodation area in which the electrode holder is mounted. When there is one electrode holder, the electrode holder is a first electrode holder 22 or a second electrode holder 23; when there are at least two electrode holders, the first electrode holder 22 and the second electrode holder 23 are used in combination, and the first electrode holder 22 is disposed below the second electrode holder 23 and snap-fitted with the base 21. The first electrode holder 22 is used to mount the stimulating electrode and the second electrode holder 23 is used to mount the collecting electrode.

Multiple second clamping jaws 223 are disposed on the first electrode holder 22 and the multiple second clamping jaws 223 can be snap-fitted with the second electrode holder 23 covered thereon so as to realize the fixed mounting of the base 21 and the electrode holder at bottom and the fixed mounting of the electrode holder at bottom and the electrode holder thereon. Multiple electroder holders are stacked up and down so that the electrode holders can be placed or removed based on actual requirements, so as to improve the entire flexibility and generality. Also, The base 21 and the electrode holders are both made from a deformable plastic material so that the multiple electrode holders can be mounted into the base 21 by pressing.

Optionally, the first clamping jaws 214, for example, are not limited to four and evenly distributed on the circumferential side of the base 21; the second clamping jaws 223, for example, are not limited to four and evenly distributed on the circumferential side of the first circular portion 221.

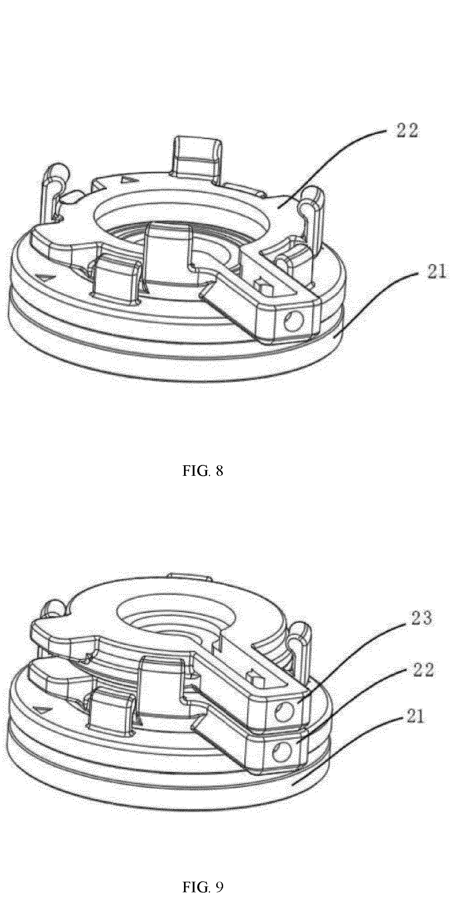

As shown in FIGS. 7 to 9, the electrode holder and the base 21 can be freely combined by stacking. Herein, the electrode holder mainly includes the first electrode holder 22 and the second electrode holder 23. When there is one electrode holder, the electrode holder is the first electrode holder 22 or the second electrode holder 23; the first electrode holder 22 can be combined with the base 21 to form an electrode holder structure with combined use of the stimulating electrode and the collecting electrode; the second electrode holder 23 can also be combined with the base 21 to form another electrode holder structure with combined use of the stimulating electrode and the collecting electrode. Of course, when there are at least two electrode holders, the electrode holders are used with combination of the first electrode holder 22 and the second electrode holder 23, and the first electrode holder 22 is disposed below the second electrode holder 23 and snap-fitted with the base 21, that is, the first electrode holder 22 and the second electrode holder 23 can be stacked and combined with the base 21 to form a third electrode holder structure with combined use of the stimulating electrode and the collecting electrode. It should be noted that the bottom of the third electrode holder structure with combined use of the stimulating electrode and the collecting electrode is the base 21, and the first electrode holder 22 is disposed on the base 21, and then the second electrode holder 23 is disposed on the first electrode holder 22. When the first electrode holder 22 and the second electrode holder 23 are used simultaneously, they must be mounted in sequence to achieve fool-proofing and avoid improper use resulting from staggered placement. Since multiple second clamping jaws 23 are disposed around the first electrode holder 22, the multiple clamping jaws on the first electrode holder 22 can be snap-fitted with the second electrode holder 23 so as to ensure mounting stability of the base 21, the first electrode holder 22 and the second electrode holder 23. The first electrode holder 22, for example, is not limited to mounting the stimulating electrode and the second electrode holder 23, for example, is not limited to mounting the collecting electrode.

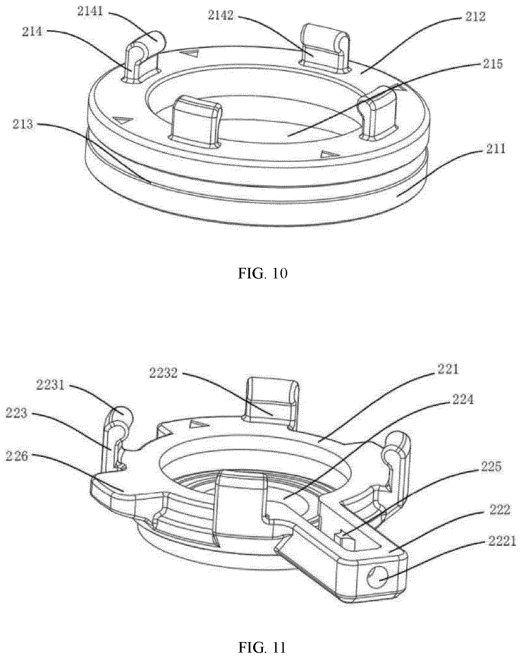

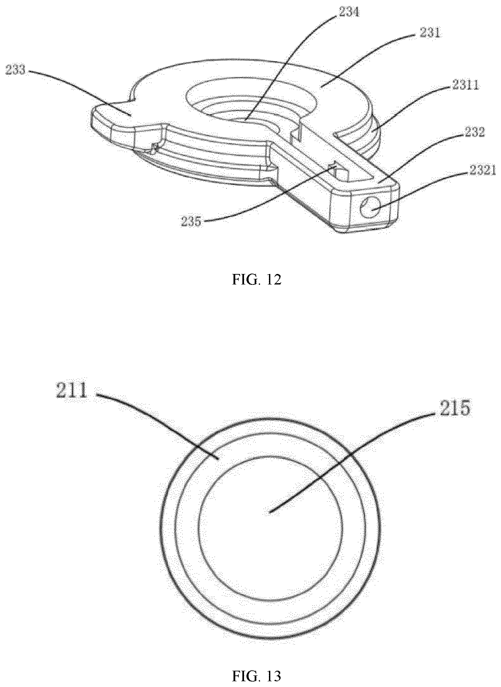

As shown in FIGS. 10 and 13, the base 21 is set to a circular shape and includes an upper-layer circular sheet 212 and a lower-layer circular sheet 211; a mounting gap 213 is disposed between the upper-layer circular sheet 212 and the lower-layer circular sheet 211 to help fit with the EEG cap. Furthermore, multiple first clamping jaws 214 are disposed circumferentially on the upper-layer circular sheet 212 and the multiple first clamping jaws 214 are spaced at a same angle, preferably, the first clamping jaws 214 are spaced at an angle of 25°, namely, four first clamping jaws 214 are disposed circumferentially on the base 21. The four first-clamping jaws 214 are formed integrally with the upper-layer circular sheet 212 and the lower-layer circular sheet 211, so that the first clamping jaws 214 can be snap-fitted with the second electrode holder 23 circumferentially, ensuring the mounting stability of the electrode holder thereon. Further, a first protrusion 2141 is disposed at an end of the first clamping jaws 214, and the first protrusion 2141 is disposed on an inner side of the first clamping jaws 214 while facing toward the center of circle of the base 21. The first protrusions 2141 can be abutted against the top of the electrode holder mounted therein so as to limit the electrode holder mounted therein. Furthermore, a first clamping groove 2142 is formed between the first protrusion 2141 and the body of the base 21, and multiple first clamping grooves 2142 of multiple first clamping jaws 214 are at a same height plane so as to form an accommodation area for placing the first electrode holder 22 or the second electrode holder 23, and the accommodation area formed is horizontal to help mount the first electrode holder 22 or the second electrode holder 23. In addition, since the base 21 is designed with four clamping jaws evenly distributed, when the first electrode holder 22 or the second electrode holder 23 is mounted thereon, the mounting direction of the first electrode holder 22 or the second electrode holder 23 can be arbitrarily switched, helping the combination designing of multiple electrode holders and improving the flexibility of wire arrangement.

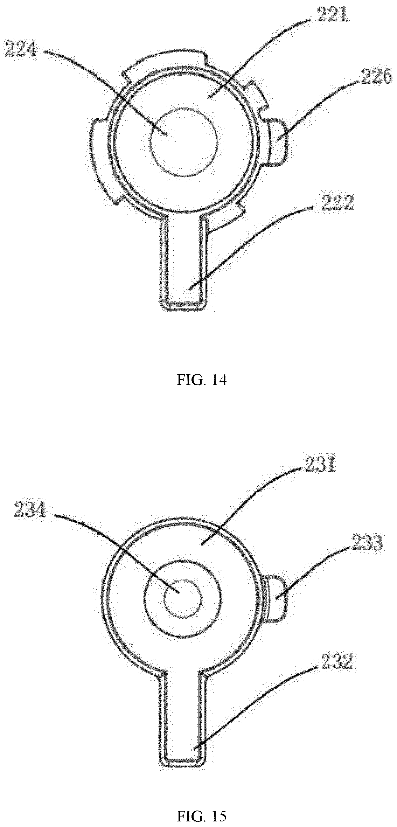

As shown in FIGS. 11 and 14, the first electrode holder 22 includes a first circular portion 221 and a first tail portion 222, the first tail portion 222 is extended and formed in a radial direction of the first circular portion 221, and the side of the first circular portion 221 extends outwardly to form multiple stop strips 226. Furthermore, multiple second clamping jaws 223 are disposed axially on the first circular portion 221, and a second protrusion 2231 facing toward the center of circle is disposed on the top of multiple second clamping jaws 223. Besides, the second clamping jaws 223 and the first clamping jaws are staggeredly distributed. A second clamping groove 2232 for accommodating the second electrode holder 23 is formed between the second protrusions 2231 and the body of the first circular portion 221. An opening is disposed at the bottom of the first circular portion 221 and a step surface is disposed around the opening to help an operator to place an electrode pad from the bottom. The bottom of the first tail portion 222 is sealed, and a first hole 2221 is opened on a top end surface of the first tail portion 222. The first hole is in communication with a fitting groove formed by the recess of the first tail portion 222 to help lead out a lead wire of the electrode pad from the first tail portion 222. In addition, a first clamping strip 225 is further disposed in the fitting groove formed by the recess of the first tail portion 222, and the lead wire of the electrode pad leading out from the first tail portion 222 can be clamped by the first clamping strip 225, helping straighten out the lead wire. Furthermore, the middle part of the first circular portion 221 is recessed to be hollow, and the hollowed area is the second through hole 224. When the first electrode holder 22 is mounted on the base 21, the first through hole 215 formed in the middle part of the base 21 is concentric with the second through hole 224 formed on the first electrode holder 22, and the first through hole 215 and the second through hole 224 are filled with conductive paste to be in contact with the electrode pad.

As shown in FIGS. 12 and 15, the second electrode holder 23 includes a second circular portion 231 and a second tail portion 232 extending out in a radial direction of the second circular portion 231. The upper and lower end surfaces of the second circular portion 231 are both plane and the side of the second circular portion 231 extends outwardly to form multiple stop strips 233. The bottom of the second circular portion 231 extends outwardly to form a flange 2311. The disposal of the flange 2311 helps the second electrode holder 23 to be clamped in the accommodation area formed by multiple first clamping grooves 2142 on the first electrode holder 22. In addition, the middle part of the second circular portion 231 is recessed inwardly to form a third through hole 234, and the recessed area also extends to the second tail portion 232. Furthermore, a second hole 2321 for leading out a lead wire of the electrode pad is opened on an end surface of the second tail portion 232, and the second hole 2321 is in communication with the recessed area of the second tail portion 232. A second clamping strip 235 is disposed in the recessed area of the second tail portion 232. The disposal of the second clamping strip 235 can clamp the lead wire of the electrode pad leading out from the second tail portion 232.

Specifically, since the first through hole 215, the second through hole 224 and the third through hole 234 are respectively opened on the base 21, the first electrode holder 22 and the third electrode holder 23, when the first electrode holder 22 and the third electrode holder 23 are sequentially stacked on the base 21, the diameter of the first through hole 215 is larger than the diameter of the second through hole 224 which is larger than the diameter of the third through hole 234 and the first through hole 215, the second through hole 224 and the third through hole 234 are are disposed concentrically. When multiple kinds of electrode holders are stacked, the conductive paste is filled to be cone-shaped, so as to improve the conductivity of multiple stacked electrode holders .

Only when the electrode holder with multiple second clamping jaws is mounted to the base can other electrode holders be mounted thereon. So, with the above disposal, the placement sequence of the electrode holders can be limited, effectively preventing the mistake of placement sequence.

The EEG cap of the present disclosure employs the electrode holder structure with combined use of the stimulating electrode and the collecting electrode, and therefore, the cap has the above advantages but is not limited to use in EEG collection or transcranial electrical stimulation.

In conclusion, the present disclosure provides an electrode holder structure, which includes a base, where multiple clamping jaws are disposed around the base, thereby enclosing an accommodation area; an electrode holder, where the electrode holder is disposed in the accommodation area, and fixedly clamped by multiple clamping jaws to form the electrode holder structure. Multiple clamping jaws are disposed circumferentially around the base, and at least one electrode holder is disposed in the multiple layers of accommodation areas formed by multiple clamping jaws, so as to realize stacking disposal of the electrode holder and the seat and improve the entire generality.

In the several embodiments provided by the present disclosure, it should be understood that the disclosed apparatus and method can be implemented another way. The above apparatus embodiments are merely illustrative, for example, the flowcharts or block diagrams in the drawings show possible system architectures, functions and operations of the apparatus, method, and computer program product in the several embodiments provided by the present disclosure. Thus, each block in the flowcharts or block diagrams may represent one module, one program fragment or one part of codes. The module, the program fragment or the part of codes includes one or more executable instructions for implementing the specified logic functions. It should be noted that in some alternative embodiments, the functions indicated in the blocks may also be performed in a sequence different from that indicated in the drawings. For example, two continuous blocks can be actually performed basically in parallel, and sometimes may be performed in a reverse sequence, which is dependent on the functions involved. It is further noted that each block in the block diagrams and/or flowcharts and the combinations of the blocks in the block diagrams and/or flowcharts may be implemented by a dedicated hardware-based system for executing specified functions or actions, or by combination of dedicated hardware and computer instructions.

Furthermore, the functional modules in the embodiments of the present disclosure can be integrated into one independent part, or exist as separate modules or two or more of the modules are integrated into one independent part.

In the description of the present disclosure, it is to be understood that orientations or positional relationships indicated by terms such as "center", "upper", "lower", "left", "right", "vertical", "horizontal", "inside", "outside", and the like are based on orientations or positional relationships shown in the drawings and are used only for convenience and simplification of descriptions of the present disclosure, rather than indicating or implying that the indicated apparatus or element shall have a specific orientation and be configured or operated in a specific orientation. Thus, the terms shall not be understood as limiting of the present disclosure. Furthermore, the terms "first", "second" and "third" are only used for the purpose of descriptions and shall not be understood as indicating or implying any relative importance.

Inspired by the ideal embodiments of the present disclosure, those skilled in the arts can make various changes and modifications based on the above descriptions within the scope of the technical idea of the present disclosure. The technical scope of the present disclosure is not limited to the contents of the above description but shall be determined by the scope of the appended claims.

## Claims

1. An electrode holder structure, comprising:
a base, wherein multiple clamping jaws are disposed circumferentially, thereby enclosing an accommodation area;
an electrode holder, disposed in the accommodation area and fixedly clamped by multiple clamping jaws to form the electrode holder structure.

2. The electrode holder structure of claim 1, wherein,
when there is one electrode holder;
the base further comprises a seat and the clamping jaws are circumferentially disposed on an edge of the seat;
one clamping groove is disposed on an inner side of each clamping jaw, and a single-layer accommodation area is formed among the clamping grooves to fit with a corresponding electrode holder.

3. The electrode holder structure of claim 1, wherein,
when there are at least two electrode holders,
and the electrode holders are either both stimulating electrode holders or collecting electrode holders; or,
in a case of combination of the stimulating electrode holder and the collecting electrode holder, the stimulating electrode holder and the collecting electrode holder are sequentially disposed above the base.

4. The electrode holder structure of claim 1, wherein,
the base further comprises a seat and and the clamping jaws are circumferentially disposed on an edge of the seat;
multiple clamping grooves are further disposed up and down on an inner side of the clamping jaws, and multiple layers of corresponding accommodation areas are formed among
multiple corresponding clamping grooves to stack and clamp corresponding electrode holders up and down.

5. The electrode holder structure of claim 31, wherein,
in a case of combination of the stimulating electrode holder and the collecting electrode holder, the stimulating electrode holder and the collecting electrode holder are sequentially disposed above the base, namely,
the clamping jaws of the base are provided with clamping grooves for forming a lower-layer accommodation area to clamp the corresponding stimulating electrode holder; and,
the stimulating electrode holder is provided with multiple clamping jaws and a protrusion is disposed on an inner side of the multiple clamping jaws to form clamping grooves for forming an upper-layer accommodation area so as to clamp the corresponding collecting electrode holder.

6. The electrode holder structure of claim 1, wherein,
a middle part of the base is hollowed to form a first through hole with a diameter of φ₁;
a middle part of the electrode holder in the lower-layer accommodation area is hollowed to form a second thorough hole A with a diameter of φₐ;
a middle part of the electrode holder in the upper-layer accommodation area is hollowed to form a third thorough hole B with a diameter of φ_{b}; wherein,
the first through hole, the second through hole and the third through hole are all concentric, and φ₁>φₐ>φ_{b}.

7. The electrode holder structure of claim 41, wherein,
the seat comprises multiple layers of stacked circular sheets, and,
a mounting gap for fitting with an electroencephalogram (EEG) cap is disposed between the multiple layers of circular sheets.

8. The electrode holder structure of claim 1, wherein,
the electrode holder comprises a circular portion and a tail portion; wherein,
a top of the circular portion is disposed as open; multiple avoiding portions are further disposed on a side of the circular portion, and each of the avoiding portions is a flat planar structure; and,
the circular portion extends outwardly in a radial direction to form the tail portion, and the tail portion extends out from a gap between adjacent clamping jaws.

9. The electrode holder structure of claim 8, wherein,
a top of the tail portion is provided with a cover plate, and the cover plate is connected with a shell of the tail portion to form a channel structure, and,
the electrode holder is a hollow shell.

10. The electrode holder structure of claim 1, wherein,
the base comprises an upper-layer circular sheet and a lower-layer circular sheet; wherein,
a mounting gap is disposed between the upper-layer circular sheet and the lower-layer circular sheet.

11. The electrode holder structure of claim 1, wherein,
the multiple clamping jaws are disposed in a spacing of same angle, and the clamping jaws corresponding to the upper-layer and low-layer accommodation areas are arranged in a staggered manner.

12. The electrode holder structure of claim 1, wherein,
the electrode holder comprises a circular portion and a tail portion; wherein,
the circular portion extends outwardly in a radial direction to form the tail portion, a bottom of a side of the circular portion extends outwardly to form a flange, and the flange is fitted with the clamping grooves in the accommodation area; and,
the side of the circular portion extends out to form multiple stop strips, an opening is disposed at the bottom of the circular portion, and a step surface is disposed around the opening.

13. The electrode holder structure of claim 1, wherein,
the electrode holder further comprises a recessed area;
the recessed area is in a middle part of the circular portion and extends to the tail portion, and,
the tail portion is provided with a hole which is in communication with the recessed area; and,
a clamping strip is further disposed in the recessed area of the tail portion.

14. The electrode holder structure of claim 1, wherein,
the base and the electrode holder are respectively made from a deformable plastic material.

15. An electroencephalogram cap, wherein,
comprising the electrode holder structure of any one of claims 1 to 14.
